(19) Europäisches Patentamt
European Patent Office
Office européen des brevets

(11) **EP 1 539 899 B1**

(12) **EUROPEAN PATENT SPECIFICATION**

(45) Date of publication and mention
of the grant of the patent:
**17.12.2008 Bulletin 2008/51**

(51) Int Cl.:
**C09J 153/00** (2006.01)     **C09J 153/02** (2006.01)
**A61F 13/15** (2006.01)

(21) Application number: **03739252.9**

(22) Date of filing: **19.06.2003**

(86) International application number:
**PCT/US2003/019658**

(87) International publication number:
**WO 2004/005418 (15.01.2004 Gazette 2004/03)**

(54) **ELASTOMERIC ADHESIVE COMPOSITIONS AND LAMINATES**

ELASTOMERE KLEBMITTEL UND LAMINATE

COMPOSITIONS ADHESIVES ELASTOMERES ET STRATIFIEES

(84) Designated Contracting States:
**AT BE BG CH CY CZ DE DK EE ES FI FR GB GR HU IE IT LI LU MC NL PT RO SE SI SK TR**

(30) Priority: **02.07.2002 US 187681**
             **26.12.2002 US 330001**

(43) Date of publication of application:
**15.06.2005 Bulletin 2005/24**

(73) Proprietor: **KIMBERLY-CLARK WORLDWIDE, INC.**
**Neenah WI 54956 (US)**

(72) Inventors:
• **ZHOU, Peiguang**
**Appleton, WI 54915 (US)**
• **NECULESCU, Cristian, M.**
**Neenah, WI 54956 (US)**

(74) Representative: **Davies, Christopher Robert**
**Frank B. Dehn & Co.**
**St Bride's House**
**10 Salisbury Square**
**London**
**EC4Y 8JD (GB)**

(56) References cited:
**US-A- 4 543 099**     **US-A- 4 698 242**
**US-A- 4 789 699**     **US-A- 5 536 563**

**Description**

This invention is directed to elastomeric adhesive compositions, and laminates made up of elastomeric adhesive film or strands self-adhered to one or more facing sheets.

Personal care garments often include elasticized portions to create a gasket-like fit around certain openings, such as waist openings and leg openings. Elastic laminates can be used in the manufacture of such garments to avoid complicated elastic-attachment steps during the garment manufacturing process.

One type of elastomeric laminate is a stretch-bonded laminate that includes elastic strands produced from an extruder and bonded to a facing sheet or sheets using a hot melt adhesive. Laminates including pre-made elastic strands can be processed online but require an elastic attachment adhesive with high add-on in order to reduce strand slippage. The cost of making stretch-bonded laminates can be relatively high due to the cost of the facing sheet or sheets, plus the cost of the elastic strands, plus the cost of the adhesive.

Another type of elastomeric laminate can be made using a vertical filament laminate-stretch-bonded laminate (VFL-SBL) process. However, the VFL-SBL process must be in off-line operation due to process complexity.

Elastomeric adhesive compositions are multifunctional in the sense that they function as an elastomer in a nonwoven composite while also serving as a hot melt adhesive for bonding substrates. Elastomeric adhesive compositions in the form of elastomeric adhesive films are currently recognized as suitable for use in the manufacture of personal care articles. More particularly, elastomeric adhesive compositions can be used to bond facing materials, such as spunbond, to one another while simultaneously elasticizing the resulting laminate. The resulting laminate can be used to form an elastomeric portion of an absorbent article, such as a region surrounding a waist opening and/or a leg opening.

Non-woven elastic adhesive film laminates may require high output of adhesive add-on to achieve a tension target for product application. High add-on of the film laminate may generate a bulky, thick feel and appearance, and high cost. Furthermore, the high adhesive output requirement for the film formation would make on-line processing even more difficult due to the limitation of hot melt equipment output capacity. Also, such film lamination processes are relatively complex and need more precise control than strand lamination since a film edge thinning effect may cause the film to break during stretching.

Some elastomeric adhesive compositions lose their adhesiveness when the compositions are stretched and then bonded between two nonwoven substrates. The elasticity of these elastomeric adhesive compositions (in terms of tension decay) is negatively affected when laminates including the compositions are aged at elevated temperatures, for example around 130 degrees Fahrenheit (54°C), which is commonly experienced under hot boxcar storage conditions. It appears that the poor tension and adhesion of such elastomer adhesive compositions results from the chosen base polymer, tackifier, and plasticizer chemistries as well as the unbalanced ratio of polymer to low molecular weight species in the formulation. US-A-4,698,242, US-A-4,543,099, US-A-5,536,563 and US-A-4,789,699 each disclose adhesive compositions.

There is a need or desire for an elastomeric adhesive composition that can be used to create elasticized portions of a personal care garment, wherein the composition does not result in high tension decay or delamination when used to produce elastic nonwoven laminates. There is a further need or desire for an elastic laminate having improved elastic and adhesion properties which can be made in a relatively simple process with reduced raw material usage while still providing adequate tension for product application.

SUMMARY OF THE INVENTION

The invention is characterised as set out in claim 1.

The invention is directed to elastomeric adhesive compositions, and laminates incorporating such elastomeric adhesive compositions, having superior elastic and adhesion properties. The laminates may include the elastomeric adhesive composition for bonding together two layers of spunbond, film, or other facing material. Additionally, or alternatively, the laminates may include strands of the elastomeric adhesive composition, which is hot melt-processable and can be formed on-line. The elastomeric adhesive composition can be self-adhered to one or more facing sheets without requiring additional adhesive. These laminates are particularly suitable for use in personal care product applications, medical garment applications, and industrial workwear garment applications.

The elastomeric adhesive composition includes a base polymer and a high softening point tackifier resin. The composition may also include a low softening point additive, with the low softening point additive present in an amount of between about 0% and about 20% by weight. The composition may further include an antioxidant, with the antioxidant present in an amount of between about 0.1% and about 1.0% by weight. The tackifier suitably has a softening point of at least 80 degrees Celsius and a viscosity of at least 1500 cps at 360 degrees Fahrenheit (182°C), and may include hydrocarbons from petroleum distillates, rosin, rosin esters, polyterpenes derived from wood, and/or polyterpenes derived from synthetic chemicals. The base polymer may include polystyrene-polyethylene-polypropylene-polystyrene (SEPS) block copolymer, styrene-isoprene-styrene (SIS), styrene-butadiene-styrene (SBS) block copolymer, thermoplastic polyurethane, and/or ethylene-propylene-diene (EPDM) copolymer. The base polymer is suitably present in an amount between about 30% and about 75% by weight, and the high softening point tackifier is suitably present in an amount between about 30% and about 70% by weight. The elastomeric adhesive composition suitably has a viscosity of about 5,000 to 80,000 cps at between 350 and 400 degrees Fahrenheit (176 and 204°C). The composition may also include elastomeric polymer strands incorporated therein to provide added reinforcement and elasticity.

In one embodiment, strands of the elastomeric adhesive composition can be self-adhered to one or more facing sheets, suitably between two facing sheets of spunbond, meltblown, film, or other facing material. The strands can be spaced apart on the facing sheet(s) by about 5 to about 15 strands per inch (2.54 cm), with each of the strands having a diameter or width of between about 0.1 and about 0.25 inch (0.25 and 0.63 cm). The laminates of the invention suitably have a basis weight between about 20 and about 120 grams per square meter. The laminates of the invention significantly improve the rate and extent of tension decay, as well as adhesion properties of the spunbond laminates compared to spunbond laminates including conventional elastomeric adhesive compositions. Furthermore, the elastic strand laminates of the invention require a lower output of adhesive add-on, compared to elastic film laminates, to achieve a tension target for product application which also results in less bulk and lower cost.

The invention also includes a method of making these elastomeric compositions and/or laminates. The method includes the steps of forming a solid phase composition of the base polymer and the high softening point tackifier resin, then heating the solid phase composition to form a liquid phase composition. Conventional hot melt equipment can be used to heat the elastomeric adhesive composition. A film is then formed by extruding the liquid phase composition onto a chill roll set at a temperature of between about 10 and about 50 degrees Celsius. Alternatively, strands can be formed by first extruding the liquid phase composition through a strand die and then onto the chill roll. The resulting film or strands can be peeled off the chill roll while stretching the film or strands. The film or strands can be stretched between about 200% and about 1200%, at an output of between about 20 and about 150 grams per square meter before stretching, from a slot coat die or extrusion film die or the strand die. The film or strands can be self-adhered to one or more facing materials, such as a nonwoven web or film. Tension in the elastic strand laminate can be adjusted by adjusting an add-on level of the elastomeric adhesive composition, or adjusting a stretch ratio of the strands, or varying the diameters of the strands.

In one embodiment, pre-formed elastomeric polymer strands can also be incorporated into the elastomeric adhesive compositions to form a reinforced elastomeric adhesive film. A facing material, such as a nonwoven web or film, can be laminated to one or both surfaces of the elastomeric adhesive film, with or without the elastomeric strands incorporated into the film.

With the foregoing in mind, it is a feature and advantage of the invention to provide elastomeric adhesive compositions and laminates having improved adhesion and elastic properties, which can be produced at a relatively low cost. The invention also includes methods of making such elastomeric compositions and laminates.

BRIEF DESCRIPTION OF THE DRAWINGS

Fig. 1 is a plan view of one embodiment of an elastomeric adhesive composition of the invention.

Fig. 2 is a cross-sectional view, taken along line 2-2 of Fig. 1, of another embodiment of an elastomeric adhesive composition of the invention.

Fig. 3 is a plan view of an elastomeric adhesive laminate of the invention.

Fig. 4 is a cross-sectional view, taken along line 4-4 of Fig. 3, of an elastomeric adhesive laminate of the invention.

Fig. 5 is a plan view of one embodiment of an elastic strand laminate of the invention.

Fig. 6 is a cross-sectional view, taken along line 6-6 of Fig. 5, of an elastic strand laminate of the invention.

Fig. 7 illustrates a representative process for making the elastomeric compositions and laminates of the invention.

Fig. 8 illustrates a representative process for making the elastomeric compositions and elastic strand laminates of the invention.

Fig. 9 is a schematic view of another process for making the elastomeric compositions and laminates of the invention.

Fig. 10 is a perspective view of a garment having an elastomeric laminate around the leg openings and waist opening.

Fig. 11 shows a schematic diagram of creep testing.

DEFINITIONS

Within the context of this specification, each term or phrase below will include the following meaning or meanings.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

"Elastic tension" refers to the amount of force per unit width required to stretch an elastic material (or a selected zone thereof) to a given percent elongation.

"Elastomeric" and "elastic" are used interchangeably to refer to a material or composite that is generally capable of recovering its shape after deformation when the deforming force is removed. Specifically, as used herein, elastic or elastomeric is meant to be that property of any material which, upon application of a biasing force, permits the material to be stretchable to a stretched biased length which is at least about 50 percent greater than its relaxed unbiased length, and that will cause the material to recover at least 40 percent of its elongation upon release of the stretching force. A hypothetical example which would satisfy this definition of an elastomeric material would be a one (1) inch sample of material which is elongatable to at least 1.50 inches (3.81 cm) and which, upon being elongated to 1.50 inches (3.81 cm) and released, will recover to a length of less than 1.30 inches (3.3 cm). Many elastic materials may be stretched by much more than 50 percent of their relaxed length, and many of these will recover to substantially their original relaxed length upon release of the stretching force.

"Elongation" refers to the capability of an elastic material to be stretched a certain distance, such that greater elongation refers to an elastic material capable of being stretched a greater distance than an elastic material having lower elongation.

"Film" refers to a thermoplastic film made using a film extrusion process, such as a cast film or blown film extrusion process. The term includes apertured films, slit films, and other porous films which constitute liquid transfer films, as well as films which do not transfer liquid.

"Garment" includes personal care garments, medical garments, and the like. The term "disposable garment" includes garments which are typically disposed of after 1-5 uses. The term "personal care garment" includes diapers, training pants, swim wear, absorbent underpants, adult incontinence products, feminine hygiene products, and the like. The term "medical garment" includes medical (i.e., protective and/or surgical) gowns, caps, gloves, drapes, face masks, and the like. The term "industrial workwear garment" includes laboratory coats, cover-alls, and the like.

"High softening point tackifier" refers to a tackifier having a softening point above 80 degrees Celsius, and a viscosity of at least 1500 cps at 360 degrees Fahrenheit (182°C) as measured by a ring and ball method (ASTM E-28).

"Layer" when used in the singular can have the dual meaning of a single element or a plurality of elements.

"Low softening point additive" refers to a tackifier or wax or low molecular weight polymers having a softening point below 80 degrees Celsius, and a viscosity of less than 1000 cps at 360 degrees Fahrenheit (182°C) as measured by a ring and ball method (ASTM E-28).

"Melt tank processable" refers to a composition that can be processed in conventional hot melt equipment rather than in an extruder. Hot melt equipment can be used online, such as in a diaper machine, whereas extruders are used offline due to equipment restrictions.

"Meltblown fiber" refers to fibers formed by extruding a molten thermoplastic material through a plurality of fine, usually circular, die capillaries as molten threads or filaments into converging high velocity gas (e.g., air) streams which attenuate the filaments of molten thermoplastic material to reduce their diameter, which may be to microfiber diameter. Thereafter, the meltblown fibers are carried by the high velocity gas stream and are deposited on a collecting surface to form a web of randomly dispersed meltblown fibers. Such a process is disclosed for example, in U.S. Patent 3,849,241 to Butin et al. Meltblown fibers are microfibers which may be continuous or discontinuous, are generally smaller than about 0.6 denier, and are generally self bonding when deposited onto a collecting surface.

"Nonwoven" and "nonwoven web" refer to materials and webs of material having a structure of individual fibers or filaments which are interlaid, but not in an identifiable manner as in a knitted fabric. The terms "fiber" and "filament" are used herein interchangeably. Nonwoven fabrics or webs have been formed from many processes such as, for example, meltblowing processes, spunbonding processes, air laying processes, and bonded carded web processes. The basis weight of nonwoven fabrics is usually expressed in ounces of material per square yard (osy) or grams per square meter (gsm) and the fiber diameters are usually expressed in microns. (Note that to convert from osy to gsm, multiply osy by 33.91.)

"Polymers" include, but are not limited to, homopolymers, copolymers, such as for example, block, graft, random and alternating copolymers, terpolymers, etc. and blends and modifications thereof. Furthermore, unless otherwise specifically limited, the term "polymer" shall include all possible geometrical configurations of the material. These configurations include, but are not limited to isotactic, syndiotactic and atactic symmetries.

"Softening point" refers to a material softening temperature, typically measured by a ring and ball type method, ASTM E-28.

"Spunbond fiber" refers to small diameter fibers which are formed by extruding molten thermoplastic material as filaments from a plurality of fine capillaries of a spinnerette having a circular or other configuration, with the diameter of the extruded filaments then being rapidly reduced as taught, for example, in U.S. Patent 4,340,563 to Appel et al., and U.S. Patent 3,692,618 to Dorschner et al., U.S. Patent 3,802,817 to Matsuki et al., U.S. Patents 3,338,992 and 3,341,394 to Kinney, U.S. Patent 3,502,763 to Hartmann, U.S. Patent 3,502,538 to Petersen, and U.S. Patent 3,542,615 to Dobo et al., each of which is incorporated herein in its entirety by reference. Spunbond fibers are quenched and generally not tacky when they are deposited onto a collecting surface. Spunbond fibers are generally continuous and often have average deniers larger than about 0.3, more particularly, between about 0.6 and 10.

"Strand" refers to an article of manufacture whose width is less than a film and is suitable for incorporating into a film, according to the invention. More particularly, a strand may be thread-like with a cylindrical cross-section, for example, or may be flat or ribbon-like with a rectangular cross-section, for example.

"Stretch-to-stop" refers to a ratio determined from the difference between the unextended dimension of a composite elastic material and the maximum extended dimension of a composite elastic material upon the application of a specified tensioning force and dividing that difference by the unextended dimension of the composite elastic material. If the stretch-to-stop is expressed in percent, this ratio is multiplied by 100. For example, a composite elastic material having an unextended length of 12.7cm (5 inches) and a maximum extended length of 25.4cm (10 inches) upon applying a force of 2000 grams has a stretch-to-stop (at 2000 grams) of 100 percent. Stretch-to-stop may also be referred to as "maximum non-destructive elongation".

"Thermoplastic" describes a material that softens and flows when exposed to heat and which substantially returns to a non-softened condition when cooled to room temperature.

"Thermoset" describes a material that is capable of becoming permanently cross-linked, and the physical form of the material cannot be changed by heat without the breakdown of chemical bonds.

"Vertical filament stretch-bonded laminate" or "VF SBL" refers to a stretch-bonded laminate made using a continuous vertical filament process, as described herein.

These terms may be defined with additional language in the remaining portions of the specification.

DETAILED DESCRIPTION OF THE

PRESENTLY PREFERRED EMBODIMENTS

The present invention is directed to elastomeric adhesive compositions and laminates having superior elastic and adhesion properties, and a method of making such compositions and laminates. The compositions and laminates can be incorporated into any suitable article, such as personal care garments, medical garments, and industrial workwear garments. More particularly, the elastomeric adhesive compositions and laminates are suitable for use in diapers, training pants, swim wear, absorbent underpants, adult incontinence products, feminine hygiene products, protective medical gowns, surgical medical gowns, caps, gloves, drapes, face masks, laboratory coats, and coveralls.

A number of elastomeric components are known for use in the design and manufacture of such articles. For example, disposable absorbent articles are known to contain elasticized leg cuffs, elasticized waist portions, and elasticized fastening tabs. The elastomeric adhesive compositions and laminates of this invention may be applied to any suitable article to form such elasticized areas.

An elastomeric adhesive composition of the invention includes a base polymer and a high softening point tackifier resin. The composition may also include a low softening point additive and/or an antioxidant. The choice of polymer and tackifier is important, as is the ratio of polymer or copolymers to tackifier. Another important consideration is the ratio of low softening point additive to high softening point tackifier.

The base polymer suitably has a styrene content of between about 15% and about 45%, or between about 18% and about 30%, by weight of the base polymer. The base polymer may achieve the styrene content either by blending different polymers having different styrene co-monomer levels or by including a single base polymer that has the desired styrene co-monomer level. Generally, the higher the styrene co-monomer level is, the higher the tension is.

The base polymer may include polystyrene-polyethylene-polypropylene-polystyrene (SEPS) block copolymer, styrene-isoprene-styrene (SIS), styrene-butadiene-styrene (SBS) block copolymer, thermoplastic polyurethane, ethylene-propylene-diene (EPDM) copolymer, as well as combinations of any of these. One example of a suitable SEPS copolymer is available from Kraton Polymers of Belpre, Ohio, under the trade designation KRATON® G 2760. One example of a suitable SIS copolymer is available from Dexco, a division of Exxon-Mobil, under the trade designation VECTOR™. Suitably, the composition includes the base polymer in an amount between about 30% and about 75% by weight of the composition.

The base polymer suitably has a Shore A hardness of between about 20 and about 90, or between about 30 and about 80. Shore A hardness is a measure of softness, and can be measured according to ASTM D-5.

In one embodiment of the invention, the base polymer may have a melt flow rate between about 5 and about 200 grams per minute, Shore A hardness between about 20 and about 70, and may be stretched up to about 1300%.

The tackifier may include hydrocarbons from petroleum distillates, rosin, rosin esters, polyterpenes derived from wood, polyterpenes derived from synthetic chemicals, as well as combinations of any of these. A key element of the compositions of the invention is a high softening point tackifier. An example of a suitable high softening point tackifier is available from Hercules Inc. of Wilmington, Delaware, under the trade designation PICOLYTE™ S115. Suitably, the composition includes the high softening point tackifier in an amount between about 30% and about 70% by weight of the composition.

A low softening point additive may be included in the compositions as well. A low softening point additive typically has a softening point below 80 degrees Celsius and a viscosity or less than 1000 cps at 360 degrees Fahrenheit (182°C), while a high softening point tackifier typically has a softening point above 80 degrees Celsius and a viscosity of at least 1500 cps at 360 degrees Fahrenheit (182°C). The use of predominantly high softening point tackifiers with high viscosity is important for adhesion improvement due to enhanced cohesive strength. However, the inclusion of relatively low amounts of low softening point additives provides instantaneous surface tackiness and pressure sensitive characteristics as well as reduced melt viscosity. Suitably, the low softening point additive is present in the composition in an amount between about 0% and about 20% by weight of the composition. One example of a particularly suitable low softening point additive is PICOLYTE™ S25 tackifier, available from Hercules Inc., having a softening point in a range around 25 degrees Celsius, or paraffin wax having a melting point of about 65 degrees Celsius may also be used.

Additionally, an antioxidant may be included in the composition, suitably in an amount between about 0.1% and about 1.0% by weight of the composition. One example of a suitable antioxidant is available from Ciba Specialty Chemicals under the trade designation IRGANOX™ 1010.

Viscosity of the formulated elastomeric adhesive composition is suitably in the range of 5,000 to 80,000 cps at 350 to 400 degrees Fahrenheit (177 and 204°C), or 10,000 to 50,000 cps at between 350 and 385 degrees Fahrenheit (177 and 196°C). The adhesive composition can be processed by conventional hot melt equipment.

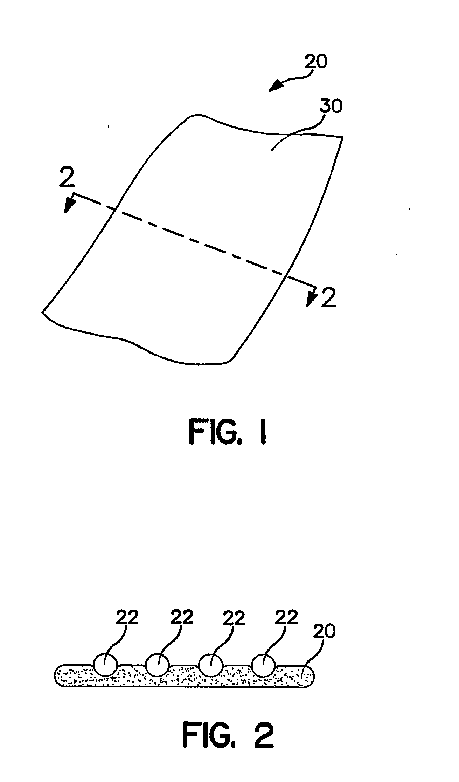

One embodiment of an elastomeric adhesive composition 20 of the invention is shown in Fig. 1. In another embodiment of the invention, shown in Fig. 2 as a cross-sectional view of Fig. 1, elastomeric reinforcing strands 22 can be adhered to and partially embedded in the elastomeric adhesive composition 20 to further enhance laminate tension control.

Materials suitable for use in preparing the elastic reinforcing strands 22 include diblock, triblock, tetrablock, or other multi-block elastomeric copolymers such as olefinic copolymers, including styrene-isoprene-styrene, styrene-butadiene-styrene, styrene-ethylene/butylenes-styrene, or styrene-ethylene/propylene-styrene, which may be obtained from Kraton Polymers, under the trade designation KRATON® elastomeric resin; polyurethanes, including those available from E. I. Du Pont de Nemours Co., under the trade name LYCRA® polyurethane; polyamides, including polyether block amides available from Ato Chemical Company, under the trade name PEBAX® polyether block amide; polyesters, such as those available from E. I. Du Pont de Nemours Co., under the trade name HYTREL® polyester; and single-site or metallocene-catalyzed palyolefins having density less than about 0.89 grams/cubic centimeter, available from Dow Chemical Co. under the trade name AFFINITY®.

A number of block copolymers can also be used to prepare the elastic reinforcing strands 22 used in this invention. Such block copolymers generally include an elastomeric midblock portion B and a thermoplastic endblock portion A. The block copolymers may also be thermoplastic in the sense that they can be smelted, formed, and resolidified several times with little or no change in physical properties (assurning a minimum of oxidative degradation). Alternatively, the elastic strands 22 can be made of a polymer that is not thermally processable, such as LYCRA® spandex, available from E. I. Du Pont de Nemours Co., or cross-linked natural rubber in film or fiber form. Thermoset polymers and polymers such as spandex, unlike the thermoplastic polymers, once cross-linked cannot be thermally processed, but can be obtained on a spool or other form and can be stretched and applied to the strands in the same manner as thermoplastic polymers. As another alternative, the elastic strands 22 can be made of a thermoset polymer, such as AFFINITY®, available from Dow Chemical Co., that can be processed like a thermoplastic, i.e. stretched and applied, and then treated with radiation, such as electron beam radiation, gamma radiation, or UV radiation to cross-link the polymer, or use polymers that have functionality built into them such that they can be moisture-cured to cross-link the polymer, thus resulting in a polymer and the enhanced mechanical properties of a thermoset.

Endblock portion A may include a poly(vinylarene), such as polystyrene. Midblock portion B may include a substantially amorphous polyolefin such as polyisoprene, ethylene/propylene polymers, ethylene/butylenes polymers, polybutadiene, and the like, or mixtures thereof.

Suitable block copolymers useful in this invention include at least two substantially polystyrene endblock portions and at least one substantially ethylene/butylenes and isoprene or butadiene mid-block portion. A commercially available example of such a linear block copolymer is available from Kraton Polymers under the trade designation KRATON® G1657 elastomeric resin. Another suitable elastomer is KRATON® G2760.

The elastic reinforcing strands 22 may also contain blends of elastic and inelastic polymers, or of two or more elastic polymers, provided that the blend exhibits elastic properties. In one embodiment, the elastic reinforcing strands 22 may be made of the same material as the elastomeric adhesive composition but in the form of elongated, rectangular strips produced from a film extrusion die having a plurality of slotted openings.

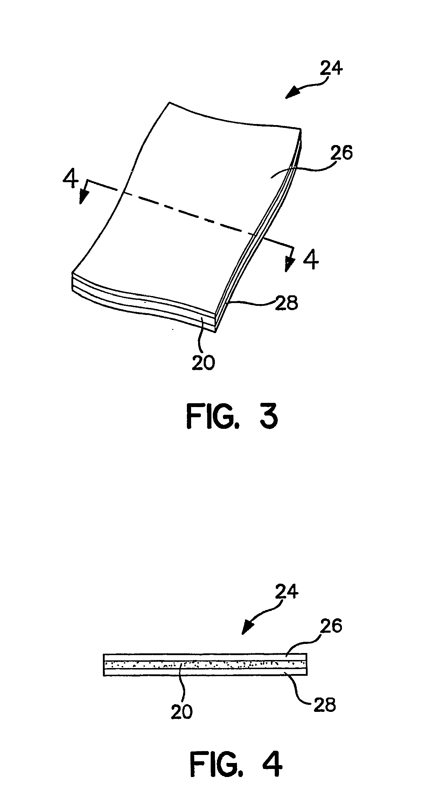

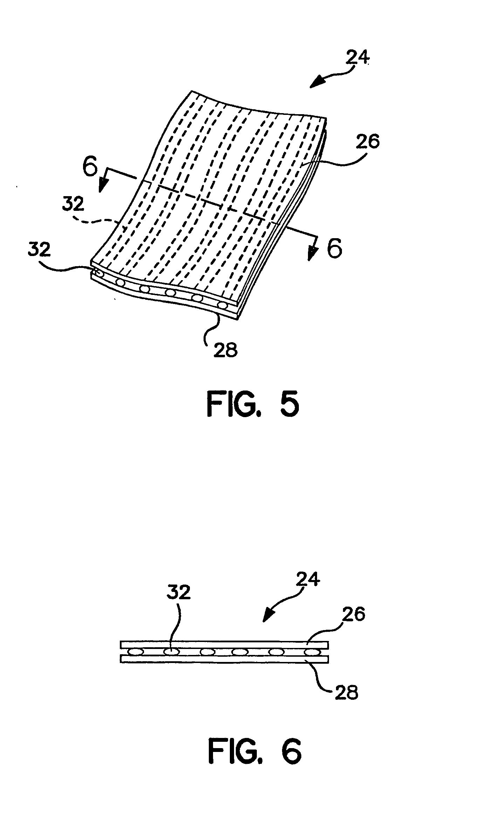

Laminates 24 of the invention may include the elastomeric adhesive composition 20 self-adhered to at least one facing sheet, or between a first facing sheet 26 and a second facing sheet 28, as shown in Fig. 3. A cross-sectional view of the laminate 24 in Fig. 3 is shown in Fig. 4. The elastomeric adhesive composition 20 may be laminated with or without the elastic reinforcing strands 22 attached to it. Alternatively, the elastomeric adhesive composition 20 itself may be in the form of elastomeric strands 32 rather than sheet form 30, as shown in Figs. 5 and 6. The elastomeric adhesive composition 20, whether in the form of sheets 30 or strands 32, is capable not only of introducing a degree of elasticity to facing materials but is also capable of providing a construction adhesive function. That is, the composition 20 adheres together the facing materials or other components with which it is in contact. It is also possible that the composition 20 does not constrict upon cooling but, instead, tends to retract to approximately its original dimension after being elongated during use in a product.

When the elastomeric adhesive composition 20 is in the form of strands 32, the strands may be laid out periodically, non-periodically, and in various spacings, groupings, and sizes, according to the effect desired from the laminate 24 and the use to which it is put. For example, the strands 32 may be spaced apart to between about 5 and about 15 strands per inch (2.54 cm). Similarly, when the elastomeric adhesive composition 20 is in sheet form 30 with elastic reinforcing strands 22 incorporated therein, the elastic reinforcing strands 22 may also be laid out periodically, non-periodically, and in various spacings, groupings, and sizes, according to the effect desired from the laminate 24 and the use to which it is put.

Both the elastomeric adhesive strands 32 and the elastic reinforcing strands 22 are substantially continuous in length. The strands 22, 32 may have a circular cross-section, but may alternatively have other cross-sectional geometries such as elliptical, rectangular as in ribbon-like strands, triangular or multi-lobal. Each strand 22, 32 suitably has a diameter between about 0.1 and about 0.25 inch (0.25 and 0.63 cm), with "diameter" being the widest cross-sectional dimension of the strand.

The facing materials 26, 28 in the laminates may be nonwoven webs or polymer films formed using conventional processes, including the spunbond and meltblowing processes described in the DEFINITIONS. For example, the facing sheets 26, 28 may each include a spunbonded web having a basis weight of about 0.1-4.0 ounces per square yard (osy), suitably 0.2-2.0 osy, or about 0.4-0.6 osy (1 osy = 34 gsm). The facing sheets 26, 28 may include the same or similar materials or different materials.

If the facing sheets 26, 28 are to be applied to the composition 20 and/or strands 22 without first being stretched, the facing sheets may or may not be capable of being stretched in at least one direction in order to produce an elasticized area. For example, the facing sheets could be necked, or gathered, in order to allow them to be stretched after application of the composition and/or strands. Various post treatments, such as treatment with grooved rolls, which alter the mechanical properties of the material, are also suitable for use. The laminate 24 suitably has a basis weight between about 20 and about 120 grams per square meter.

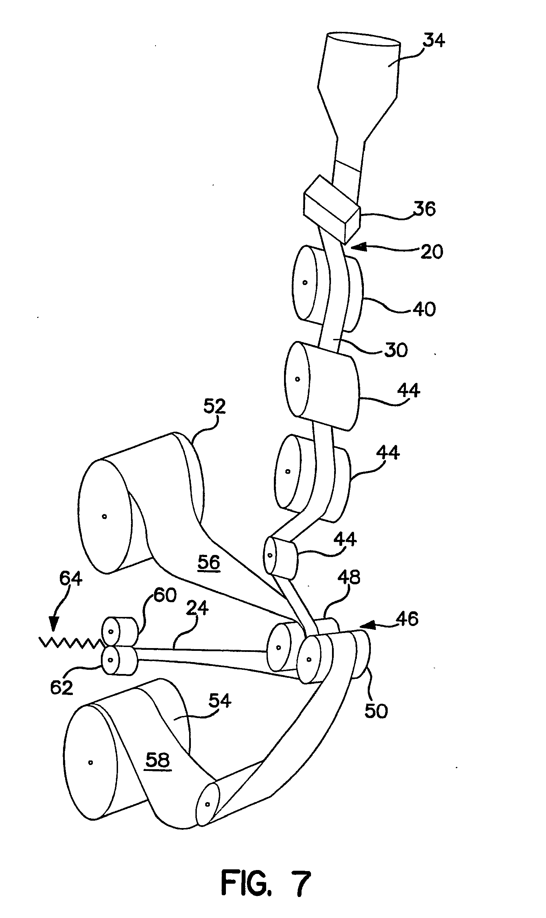

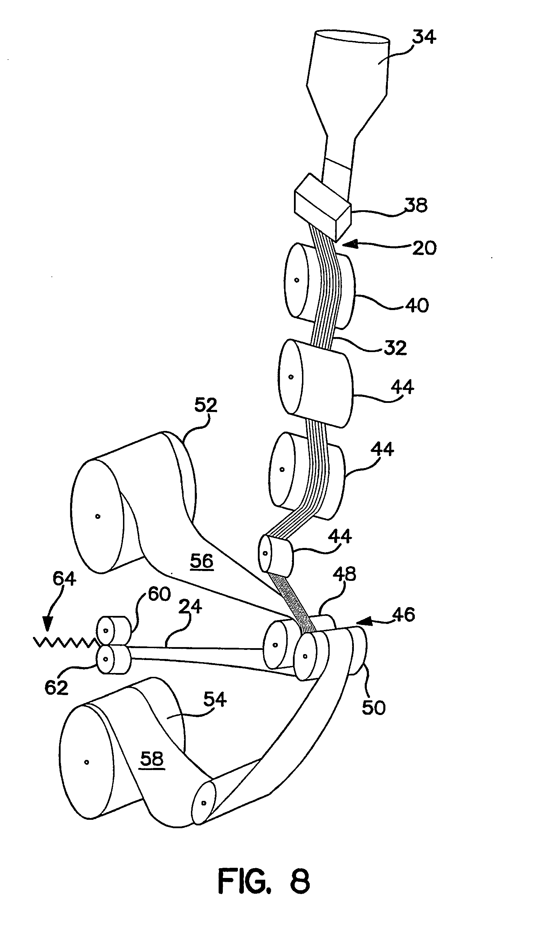

Figs. 7 and 8 illustrate a method and apparatus for making an elastomeric adhesive composition 20 and laminate 24 of the invention. While Figs. 7 and 8 illustrate a composite VF SBL process it will be appreciated that other processes consistent with the invention may be used.

The elastomeric adhesive composition 20 is formulated by mixing the base polymer and the tackifier in a Sigma blade batch mixer or by other suitable compounding methods including continuous mixing processes such as twin screw extrusion, resulting in a solid phase composition. Conventional hot melt equipment can be used to heat the composition. For example, solid blocks of the composition may be heated in a melt tank 34 at about 385 degrees Fahrenheit (196°C), for example, to form a liquid phase, and then processed through a slot coat die or an extrusion film die 36 (Fig. 7) or a strand die 38 (Fig. 8) at between about 20 and about 150 grams per square meter (gsm), or between about 40 and about 100 gsm output before stretching, onto a chill roll 40 or similar device at between about 10 and about 55 degrees Celsius, for example, in the form of a sheet or ribbon 30 (Fig. 7) or multiple strands 32 (Fig. 8). Film output or strand output (gsm) denotes grams per square meter as measured by cutting the film or strands with a template and weighing them. The sheet 30 or strands 32 are then stretched (between about 200% and about 1200%) and thinned as the composition is peeled off the chill roll 40 and passed to one or more fly rollers 44 towards a nip 46. The sheet 30 or strands 32 may be stretched down to a narrower width and thinned by the fly rollers 44 during their passage to the nip 46. The nip 46 is formed by opposing first and second nip rollers 48, 50.

In film form, the composition suitably has a thickness of about 0.001 inch (0.025 mm) to about 0.05 inch (1.27 mm), alternatively of from about 0.001 inch (0.025 mm) to about 0.01 inch (0.25 mm), and a width of from about 0.05 inch (1.27 mm) to about 10 inches (25.4 cm), alternatively of from about 0.5 inch (1.27 cm) to about 5 inches (12.7 cm). The elastomeric adhesive film 30 may also be capable of imparting barrier properties in an application.

In an embodiment in which the composition 20 is in the form of strands 32, the configuration of the strand die 38 determines the number of strands, diameter of the strands, spacing between the strands, as well as shape of the strands.

The elastomeric adhesive composition in the form of either strand or film laminate suitably has an elongation of at least 50 percent, alternatively of at least 150 percent, alternatively of from about 50 percent to about 500 percent, and a tension force of less than about 400 grams force per inch (2.54 cm) width, alternatively of less than about 275 grams force per inch (2.54 cm) width, alternatively of from about 100 grams force per inch (2.54 cm) width to about 250 grams force per inch (2.54 cm) width. Tension force, as used herein, is determined one minute after stretching the elastic strand laminate to 100% elongation.

In order to form a laminate 24 of the invention, first and second rolls 52 and 54, respectively, of spunbond facing material 56, 58 or other nonwoven or film are fed into the nip 46 on either side of the elastomeric composition 20 and are bonded by the adhesive present in the composition. The facing material 56, 58 might also be made in situ rather than unrolled from previously made rolls of material. While illustrated as having two lightweight gatherable spunbond facings 56, 58, it will be appreciated that only one facing material, or various types of facing materials, may be used. The elastomeric composite laminate 24 can be maintained in a stretched condition by a pair of tensioning rollers 60, 62 downstream of the nip 46 and then relaxed as at Ref. No. 64 (Figs. 7 and 8).

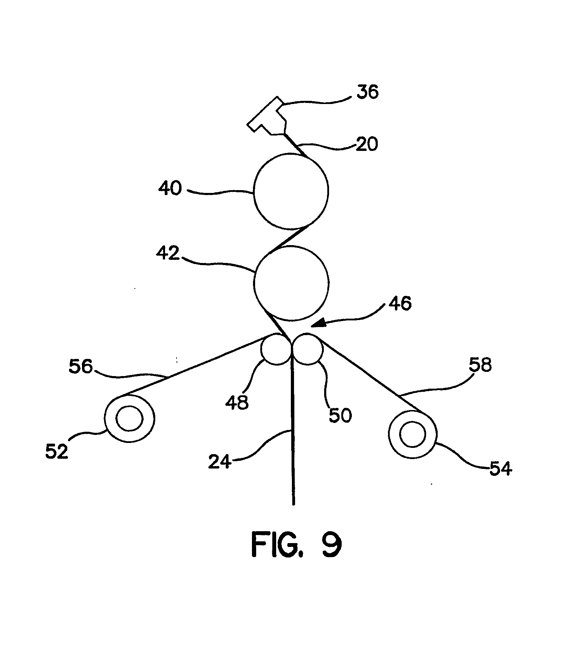

Fig. 9 illustrates a VF SBL process in which no fly rollers 44 are used. Instead, the elastomeric adhesive composition is extruded onto first chill roller 40. The composition is stretched between first and second chill rollers 40 and 42 and the nip 46. Except for the lack of fly rollers, the processes of Figs. 7 and 8 are similar to the process of Fig. 9. In either case, the composition 20 can be laminated between a first facing layer 56 and a second facing layer 58 at the nip 46.

Tension within the laminate 24 may be controlled through varying the percentage stretch, or stretch ratio, of the composition prior to adhesion to the facing sheet(s), and/or through the amount of composition add-on or thickness, with greater stretch and greater add-on or thickness each resulting in higher tension. Tension can also be controlled through selection of the elastomeric adhesive composition, and/or by varying strand geometries and/or spacing between strands. For example, holes in the strand die 38 through which the composition passes to form strands may vary in diameter. The laminate of the invention suitably has tension of at least 100 grams/inch at 100% elongation, or at least 200 grams/inch at 100% elongation.

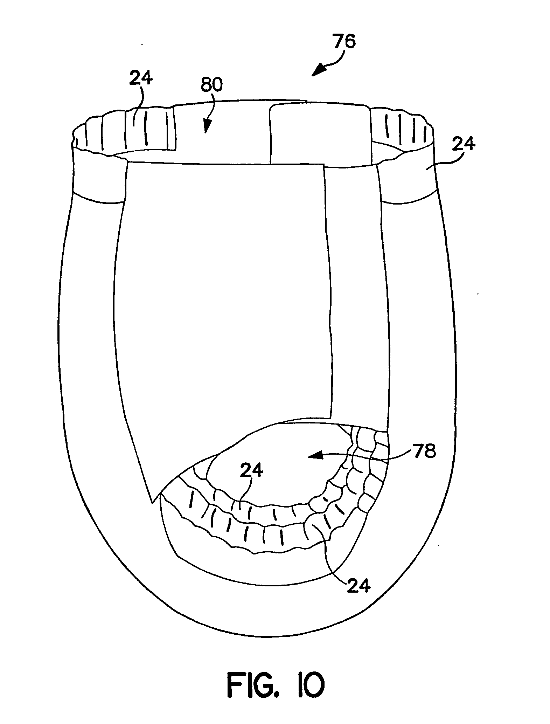

The resulting elastomeric adhesive compositions and laminates are particularly useful in providing elasticity in personal care absorbent garments 76, as shown in Fig. 10. More specifically, as shown in Fig, 10, the elastomeric adhesive laminates are particularly suitable for use in providing a gasket-like fit around leg openings 78 and waist openings 80. The laminates of this invention are less likely to undergo tension decay or delamination compared to similar laminates incorporating current commercial elastomeric adhesive compositions, as demonstrated in the examples below. Also demonstrated in the examples below is the laminate's relatively high stretch-to-stop ratio, suitably at least 50%, or at least 100%, or at least 150%, or at least 250%, and the relatively low creep, suitably less than 10% creep after aging at 100 degrees Fahrenheit for 90 minutes.

Test Methods

_Elongation_

The elongation of an elastic composite laminate according to the invention is suitably determined as follows. A 1-inch wide by 4-inch (2.54 by 10 em) long sample of the laminate is provided. The central 3-inch (7.62 cm) area of the sample is marked. The test sample is then stretched to its maximum length, and the distance between the marks is measured and recorded as the "stretched to stop length." The percent elongation is determined according to the following formula:

$$\{(\text{stretched to stop length (in inches)}) - 3\}/3 \times 100$$

If a 1-inch by 4-inch area is not available, the largest sample possible (but less than 1-inch by 4-inches) is used for testing with the method being adjusted accordingly.

_Tension Force_

The tension force of an elastic composite laminate according to the invention is determined on a test sample of the laminate having a width of 1 inch (2.54 cm) and a length of 3 inches (7.62 cm). A test apparatus having a fixed clamp and an adjustable clamp is provided. The adjustable clamp is equipped with a strain gauge commercially available from S. A. Mieier Co. under the trade designation Chatillon DFIS2 digital force gauge. The test apparatus can elongate the test sample to a given length. One longitudinal end of the test sample is clamped in the fixed clamp of the test apparatus with the opposite longitudinal end being clamped in the adjustable clamp fitted with the strain gauge. The test sample is elongated to 100 percent of its elongation (as determined by the test method set forth above). The tension force is read from the digital force gauge after 1 minute. At least three samples of the elasticized area are tested in this manner with the results being averaged and reported as grams force per inch width.

*Creeping Resistance of Elastic Strands*

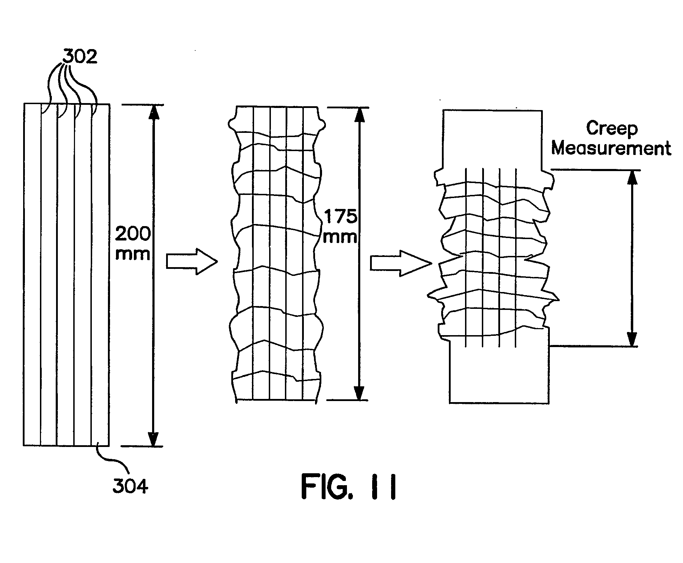

Twelve elastic strands 302, approximately 2.5 mm apart in the cross-direction and each elongated approximately 300%, were adhesively attached and sandwiched between two 4-inch wide continuous polypropylene spunbonded layers 304 to form a laminate. The laminate was fully extended by hanging a weight (about 500 grams or higher) at one end of the laminate, and a 200 mm machine-direction length was then marked. The laminate was then released, such that the 200 mm length snapped back to 175 mm, whereupon the 175 mm length was marked. The laminate was then stapled to a piece of cardboard at the 175 mm length. The marked length of the laminate was then cut to release tension in the elastic strands 302, and the snapback length of the strands was measured. An illustration of the creeping test procedure is shown in Figure 11.

Initial creep percentage was calculated by first determining the difference between the 175 mm length and the snapback length, then dividing the difference by the 175 mm length and multiplying the quotient by 100, as shown in the following equation:

$$\text{Initial Creep \%} = (175_{mm} - X_{\text{initial creep}})/175 \times 100$$

The sample was then placed in an oven at 100 degrees Fahrenheit for 90 minutes to measure aging creep. Aging creep percentage was then calculated by determining the difference between the 175 mm length and that snapback length, then dividing the difference by the 175 mm length and multiplying the quotient by 100, as shown in the following equation:

$$\text{Aging Creep \%} = (175_{mm} - Y_{\text{aged creep}})/175 \times 100$$

$X_{\text{initial}}$ creep and $Y_{\text{aged}}$ creep readings were taken from the averaged measurements of the 24 strands during the tests.

EXAMPLE 1

In this example, several formulations of the elastomeric adhesive composition used in the invention were tested for tension decay and/or adhesion.

Composition 1

In this example, the formulated elastomeric adhesive composition was made up of 55 wt% VECTOR™ 4111 SIS polymer and 45 wt% ESCOREZ™ 5340 tackifier, both available from Bxxon-Mobil. ESCOREZ™ 5340 has a softening point of 140 degrees Celsius and viscosity of 5000 cps at 177 degrees Celsius. The composition had an output of 89 gsm before stretching, and was stretched to 600%.

Adhesion of the elastomeric adhesive composition was tested by laminating the composition between two 0.6 osy (20.4 gsm) spunbond facings. A 2-inch (5.08 cm) wide sample of the laminate, tested at 50% elongation, had a green tension of 136 grams and a permanent set of 12.3%. It was found that no delamination occurred after 2 weeks of aging at 130 degrees Fahrenheit (54°C), under a laminate tension of 81.7 grams. After 2 weeks under these conditions, tension loss of the laminate was 40%, and permanent set was 15.7%.

Composition 2

Another elastomeric adhesive composition was made up of 48 wt% VECTOR™ 4111 SIS polymer, 9 wt% VECTOR™ 4411 SIS polymer, 38 wt% ESCOREZ™ 5340 tackifier, and 5 wt% paraffin wax. This composition had 120 gsm film output before stretching, and was stretched to 800%. The composition was laminated between two 0.6 osy (20.4 gsm) spunbond facings after being extruded through a film die.

An Instron tester, or Sintech tester, or similar instrument, was used to measure tension of a 2-inch (5.08 cm) sample of the laminate at room temperature. The tension in the laminate during a first cycle was determined to be 167.9 grams (g) at 50% elongation. During retraction of the first cycle, the tension in the laminate was determined to be 68.1 g. The laminate experienced a hysteresis loss of 59.4%. A permanent set for cycle 1 was 18.7%, and a permanent set for cycle 2 was 21%. High permanent set indicates poor elasticity, while low permanent set indicates good elasticity. The laminate experienced no delamination after 2 weeks at 130 degrees Fahrenheit (54°C).

Composition 3

This composition had the same formulation as the previous composition, but included ESCOREZ™ 5415 tackifier in place of ESCOREZ™ 5340 tackifier. ESCOREZ™ 5415 has a lower softening point of 118 degrees Fahrenheit (47°), and a lower viscosity of 900 cps at 177 degrees Celsius. Consequently, when tested in the same manner as the previous compositions, namely between two 0.6 osy (20.4 gsm) spunbond facings, this composition was found to have poor adhesion qualities, with the laminate delaminating in 24 hours.

Composition 4

This composition had the same formulation as the two previous composition, but included ESCOREZ™ 5320 tackifier in place of either ESCOREZ™ 5340 tackifier or ESCOREZ™ 5415 tackifier. ESCOREZ™ 5320 has a relatively low softening point of 122 degrees Celsius, and a relatively low viscosity of 1500 cps at 177 degrees Celsius. Consequently, when tested in the same manner as the previous compositions, namely between two 0.6 osy (20.4 gsm) spunbond facings, this composition was found to have poor adhesion qualities, with the laminate delaminating in 24 hours.

Composition 5

In this example, the formulated elastomeric adhesive composition was made up to 65 wt% KRATON® G 2760 SEPS copolymer, available from Kraton Polymers, and 35 wt% PICOLYTE™ S115 tackifier, available from Hercules Inc. The materials were formulated in a Sigma blade batch mixer. The Brookfield viscosity of the formulation was determined to be about 51,000 cps at 380 degrees Fahrenheit (193°C). Solid blocks of the adhesive were heated in a melt tank at 385 degrees Fahrenheit (196°C) and slot coated on a chill roll at 52 degrees Celsius. The process involved peeling off the film from the chill roll while stretching the material up to about 700% at 47 gsm output (before stretching) from the slot coat die. The stretched film was then laminated by nip roll with two nonwoven spunbond webs on each side in a continuous manner.

The laminate appeared to be soft and elastomeric. The green tension value, as measured quickly off-line was in the range of 170-190 grams/inch (2.54 cm) width. Specifically, 5 inch (12.7 cm) long specimens were clamped and elongated to 100%. The tension reading was recorded from an electronic gauge one minute after clamping. The tension remaining in the laminate was 100-110 grams/inch (2.54 cm) width after aging at 130 degrees Fahrenheit (54.4°C) for 22 hours. No delamination was observed even after aging at 130 degrees Fahrenheit (54.4°C) for an excess of 4 days.

EXAMPLE 2

A series of formulated compositions were produced to compare the effects of low softening point tackifiers versus high softening point tackifiers. A variety of tackifiers having various softening points were used in various combinations, summarized in Table 1. The tackificrs user were:

PICOLYTE™ S25, available from Hercules Inc., having a softening point of 15-25 degrees Celsius, and viscosity of 1,000 cps at 80 Degrees Celsius;
PICOLYTE™ S 115, available from Hercules Inc., having a softening point of 115 degrees Celsius, and viscosity of 10,000 cps at 150 degrees Celsius; and
STABELITE™ 5, available from Hercules Inc., having a softening point of 79 degrees Celsius.

These formulated compositions were laminated with 0.6 osy (20.4 gsm) spunbond facings and were then tested for tension decay and delamination. The tension decay was measured by first measuring the "green" tension at 100% elongation of a 2-inch wide, 5-inch long sample (5.08 by 12.7 cm). The tension reading was recorded from an electronic gauge one minute after clamping. After aging the samples at 130 degrees Fahrenheit (54.4°C) for 1 day, the "aged" tension was then measured in the same manner as the green tension and the resulting aged tension was compared to the green tension to determine whether, or to what extent, tension decay occurred.

It was found that low softening point tackifier alone in the formulation not only caused poor bonding because of low cohesion but also generated low green tension and high tension decay. Results are shown in Table 1.

Table 1: Comparison of Tackifiers in Elastic Composite Laminates

| Sample | Tackifier Composition (percentage of total tackifier included in composition) | | | Observations |
|---|---|---|---|---|
| | PICOLYTE™ S25 | PICOLYTE™ S115 | STABELITE™ 5 | |
| 1 | 100% | -- | -- | Delaminated in 10 minutes |
| 2 | -- | 100% | -- | No delamination |
| 3 | 25% | 75% | | Green tension decreased 20-40%; tension decay was more than 60% after aging at 130°F (54.4°C) for 22 hours; delaminated within 24 hours |
| 4 | 25% | | 75% | Green tension = 210 g/in width; 100% tension decay after aging at 130°F (54.4°C) for 22 hours; delaminated within 24 hours |

The results confirm that high softening point tackifiers are critical for elastomeric adhesive tension control and adhesion improved in addition to base polymer choice.

EXAMPLE 3

In this example, a self-adhering elastic strand laminate of the invention was formed by creating an elastomeric adhesive composition including 50 wt% VECTOR™ 411 SIS polymer, 10 wt% VECTOR™ 4411 SIS polymer, 39.5 wt% ESCOREZ™ 5340 tackifier, each available from Exxon-Mobil, and 0.5% IRGANOX™ 1010 antioxidant, available from Ciba Specialty Chemicals. The elastomeric adhesive composition was formulated in a Sigma blade mixer or compounded by a twin screw extruder. The Brookfield viscosity of the formulation was about 66,500 cps at 385 degrees Fahrenheit (196°C). A solid block fonn of the adhesive was heated in a melt tank at 385 degrees Fahrenheit (196°C) and extruded from a strand die onto a chill roll at 52 degrees Fahrenheit (11.1°C), stretching the strands up to about 500% at 28 gsm output. The strand die was 12 inches wide, with 12 strands per inch each having a diameter of 50 mil. The laminate was then formed by nipping the strands between two 0.5 osy (17 gsm) non-woven spundbond webs.
Initial tension in the laminate was 103 grams per square inch (6.45 cm$^2$) at 50% elongation. The laminate tension remained at 85 g/in$^2$ (6.45 cm$^2$) after aging at 130 degrees Fahrenheit (54.4°C) for a week. No delamination was observed even after aging at 130 degrees Fahrenheit (54.4°C) for a week.

EXAMPLE 4

In this example, it was shown that a strand laminate provides more tension than a film laminate. Furthermore, the strand laminates in this example showed better tension retention after aging than the film laminates. Consequently, the strand laminates provide required tension for product application at a much lower add-on level as indicated by the much higher ratio of tension (g) to film or strand output (gsm) as shown in Table 2.
Each of the laminates were tested using a 2-inch wide sample with two facings of 0.5 osy (17 gsm) PRISM bi-component material, available from Kimberly-Clark Corporation. A description of PRISM is taught in U.S. Patent No. 5,336,552 to Strack et al. Two different film laminates and two different strand laminates were formed from the elastomeric adhesive composition described in the previous example, with the samples varying in terms of output and/or stretch.
The strand laminates exhibited much lower tension decay in terms of low tension loss percentage after aging compared to the film laminates. The lower tension loss and lower tension relaxation of the strand laminates, compared to the film laminates, indicates that the strand laminates had better elastic properties. Detailed results of strand laminates and film laminates are shown in Table 2.

Table 2: Comparison of Strand and Film Laminates

| Sample Identification | Ratio of Tension (g/output gsm) | Initial Tension (g/in² (6.54 cm² at 50% elongation) | Tension after aging at 130 degrees F, one week | Tension loss % after aging | Tension relaxation % at 50%, 30 min |
|---|---|---|---|---|---|
| Film laminate (90 gsm before stretch, 700% (stretch) | 1.33 | 120 | 71 | 41 | 42 |
| Strand laminate (28 gsm before stretch, 700% stretch) | 2.61 | 73 | 63 | 14 | 36 |
| Film laminate (120 gsm before stretch, 700% stretch) | 1.14 | 137 | 81 | 41 | 40 |
| Strand laminate (28 gsm before stretch, 500% stretch) | 3.68 | 103 | 85 | 17.5 | 38 |

EXAMPLE 5

In this example, two clastic strand laminates of the invention were compared to a VFL-SBL laminate and a LYCRA laminate in terms of creep percentage and stretch-to-stop properties. Creep percentage was measured according to the test method described above. The higher stretch-to-stop ratio and lower creep percentage of the strand laminates indicates better elasticity and adhesion properties. Results are shown in Table 3.

| Sample | Note | Stretch -to-Stop % | Creep % after aging at 100 degrees F 90 min |
|---|---|---|---|
| Strand 1 | 55% VECTOR™ 4111, 45% ESCOREZ™ 5340, 800% stretch, 53 gsm output, 0.5 osy (17 gsm) spunbond facings | 230% | ~0% |
| Strand 2 | 50% VECTOR™ 4111, 10% VECTOR™ 4411, 39.5% ESCOREZ™ 5340, and 0.5% IRGANOX™ 1010, 500% stretch, 28 gsm, 0.5 osy (17 gsm) PRISM facings | 270% | <10% |
| VFL-SBL | KRATON™ 6631 using extruder, H2096 adhesive add-on 4-10 gsm, 0.5 osy (17 gsm) spunbond facings | ~170% | ~30% |
| LYCRA™ | LYCRA™ 940, strand from DuPont, H2525A adhesive, add-on 7-10 gsm, 0.5 osy (17 gsm) spundbond facings | 150-180% | 20-50% dependent on applications |

It will be appreciated that details of the foregoing embodiments, given for purposes of illustration, are not to be construed as limiting the scope of this invention.

**Claims**

1. An elastomeric adhesive composition, **characterized by**:

   a base polymer, wherein the base polymer is present in the composition in an amount between about 30% and about 75% by weight, the base polymer comprising at least one of the group consisting of polystyrene-polyethylene-polypropylene-polystyrene (SEPS) block copolymer, styrene-isoprene-styrene (SIS), styrene-butadiene-styrene (SBS) block copolymer, thermoplastic polyurethane, ethylene-propylene-diene (EPDM) copolymer, and combinations thereof;
   a high softening point tackifier resin wherein the high softening point tackifier comprises at least one type of hydrocarbon selected from the group consisting of petroleum distillates, rosin, rosin esters, polyterpenes derived from wood, polyterpenes derived from synthetic chemicals, and combinations thereof, the tackifier resin having a softening point of at least 80 degrees Celsius, and viscosity of at least 1500 cps at 360 degrees Fahrenheit (182°C), wherein the composition has a viscosity of about 5,000 to 80,000 cps at between 350 and 400 degrees Fahrenheit (177 and 204°C).

2. The composition of Claim 1, wherein the base polymer comprises a styrene content of between about 15% and about 45% by weight.

3. The composition of Claim 1 or 2, wherein the base polymer comprises a styrene content of between about 18% and about 30% by weight.

4. The composition of any preceding claim, wherein the base polymer has a melt flow rate between about 5 and about 200 grams per minute, a Shore A hardness between about 20 and about 70, and may be stretched up to about 1300%.

5. The composition of any preceding claim, wherein the high softening point tackifier is present in the composition in an amount between about 30% and about 70% by weight.

6. The composition of any preceding claim, further comprising a low softening point additive having a softening point of less than 80 degrees Celsius and a viscosity of less than 1000 cps at 360 degrees Fahrenheit (182°C), present in an amount between about 0% and about 20% by weight.

7. The composition of any preceding claim, further comprising an antioxidant in an amount between about 0.1 % and about 1.0% by weight.

8. The composition of any preceding claim, wherein the composition has a viscosity of about 10,000 to 50,000 cps at between 350 and 385 degrees Fahrenheit (177 and 196°C).

9. The composition of any preceding claim, wherein the composition is formed as a plurality of extruded strands.

10. The composition of any of Claims 1 to 8, wherein the composition is formed as an extruded film.

11. The composition of any of Claims 1 to 8, wherein the composition is formed as a combination of a plurality of extruded strands and an extruded film.

12. The composition of any preceding claim, wherein the composition can be processed by conventional hot melt equipment.

13. An elastomeric laminate, comprising:

    at least one facing sheet; and
    a melt-tank processable elastomeric adhesive composition self-adhered tu the at least one facing sheet, the elastomeric adhesive composition being as claimed in any of Claims 1 to 8.

14. The elastomeric laminate of Claim 13, wherein the composition is formed as an extruded film.

15. The elastomeric laminate of Claim 14, wherein the composition is formed as a plurality of extruded strands.

**16.** The elastomeric laminate of Claim 15, wherein the plurality of strands are spaced apart on the at least one facing sheet by about 5 to about 15 strands per inch (2.54 cm).

**17.** The elastomeric laminate of Claim 16, wherein each of the plurality of strands has a diameter between about 0.1 and about 0.25 inch (0.25 and 0.63 cm).

**18.** The elastomeric laminate of any of Claims 13 to 17, wherein the at least one facing sheet comprises a nonwoven web selected from a spunbond web and a meltblown web.

**19.** The elastomeric laminate of any of Claims 13 to 18, wherein the at least one facing sheet comprises a film.

**20.** The elastomeric laminate of any of Claims 13 to 19, wherein the laminate has a basis weight between about 20 and about 120 grams per square meter.

**21.** The elastomeric laminate of any of Claims 13 to 20, wherein the laminate exhibits a stretch-to-stop ratio of at least 50%.

**22.** The elastomeric laminate of Claim 21, wherein the laminate exhibits a stretch-to-stop ratio of at least 150%.

**23.** The elastomeric laminate of Claim 21, wherein the laminate exhibits a stretch-to-stop ratio of at least 250%.

**24.** The elastomeric laminate of any of Claims 13 to 23, wherein the laminate has tension of at least 100 grams per inch at 100% elongation.

**25.** The elastomeric laminate of any of Claims 13 to 23, wherein the laminate has tension of at least 200 grams per inch at 100% elongation.

**26.** The elastomeric laminate of any of Claims 13 to 25, wherein the laminate exhibits less than 10% creep after aging at 100 degrees Fahrenheit for 90 minutes.

**27.** The elastomeric laminate of any of Claims 13 to 26, further comprising:

a garment incorporating the elastomeric laminate into a structure of the garment.

**28.** The elastomeric laminate of Claim 27, wherein the garment is one selected from the group consisting of personal care garments, medical garments, and industrial workwear garments.

**29.** The elastomeric laminate of Claim 27, wherein the garment is one selected from the group consisting of diapers, training pants, swim wear, absorbent underpants, adult incontinence products, feminine hygiene products, protective medical gowns, surgical medical gowns, caps, gloves, drapes, face masks, laboratory coats, and coveralls.

**30.** A method of making an elastomeric adhesive composition, comprising:

forming a solid phase composition as claimed in any of Claims 1 to 8; forming a liquid phase composition by heating the solid phase composition;
forming a film by extruding the liquid phase composition onto a chill roll; and
peeling the film off the chill roll while stretching the film.

**31.** The method of Claim 30, comprising extruding the liquid phase composition through a slot coat die onto the chill roll.

**32.** The method of Claim 30 or 31, comprising stretching the film at an output of between about 50 and about 120 grams per square meter before stretching, from the slot coat die.

**33.** The method of Claim 30, 31 or 32, comprising extruding the liquid phase composition through an extrusion die onto the chill roll.

**34.** The method of any of Claims 30 to 33, comprising stretching the film at an output of between about 50 and about 120 grams per square meter, before stretching, from the extrusion die.

35. The method of any of Claims 30 to 34, comprising stretching the film up to about 1200%, preferably up to about 1000%, preferably up to about 700%, preferably up to about 500%.

36. The method of any of Claims 30 to 35, further comprising laminating the stretched film between two facing sheets.

37. The method of Claim 36, wherein at least one of the facing sheets comprises a non-woven web.

38. The method of Claim 36, wherein at least one of the facing sheets comprises a film.

39. The method of any of Claims 30 to 38, wherein the liquid phase composition is formed by heating the solid phase composition at between about 350 and about 400 degrees Fahrenheit (176 and 204°).

40. The method of any of Claims 30 to 39, wherein the chill roll is set at a temperature of between about 10 and about 50 degrees Celsius while the film is being formed.

41. The method of any of Claims 30 to 40, comprising forming the film into a plurality of extruded strands.

42. A method of making an elastic strand laminate, comprising:

heating an elastomeric adhesive composition as claimed in any of Claims 1 to 7 in hot melt equipment;
processing the elastomeric adhesive composition through a strand die onto a chill roll to form a plurality of elastic strands;
stretching the plurality of clastic strands between the chill roll and a nip roll; and
self-adhering the plurality of elastic strands to at least one facing sheet.

43. The method of Claim 42, comprising stretching the plurality of elastic strands between about 200% and about 1200%.

44. The method of Claim 42 or 43, comprising processing the elastomeric adhesive composition through the strand die at an output of between about 20 and about 150 grams per square meter before stretching.

45. The method of any of Claims 42 to 44, wherein the at least one facing sheet comprises a film.

46. The method of Claim 45, further comprising processing the film while simultaneously processing the elastomeric adhesive composition.

47. The method of any of Claims 42 to 46, wherein the at least one facing sheet comprise a nonwoven web selected from a spunbond web and a meltblown web.

48. The method of any of Claims 42 to 47, further comprising adjusting an add-on level of the elastomeric adhesive composition to vary tension in the elastic strand laminate.

49. The method of any of Claims 42 to 48, further comprising adjusting a stretch ratio of the plurality of elastic strands to vary tension in the elastic strand laminate.

50. The method of any of Claims 42 to 49, wherein the strand die comprises holes of varying diameter.

**Patentansprüche**

1. Elastomere Klebstoffzusammensetzung, **gekennzeichnet durch:**

ein Grundpolymer, wobei das Grundpolymer in einer Menge zwischen ungefähr 30 Gew.-% und ungefähr 75 Gew.-% in der Zusammensetzung vorhanden ist, wobei das Grundpolymer wenigstens eines aus der Gruppe bestehend aus Polystyrol-Polyethylen-Polypropylen-Polystyrol (SEPS) Blockcopolymer, Styrol-Isopren-Styrol (SIS), Styrol-Butadien-Styrol (SBS) Blockcopolymer, thermoplastischem Polyurethan, Ethy-len-Propylen-Dien (EPDM) Copolymer und Kombinationen daraus umfasst;
ein Klebrigmacherharz mit hohem Erweichungspunkt, wobei der Klebrigmacher mit hohem Erweichungspunkt wenigstens eine Art von Kohlenwasserstoff, ausgewählt aus der Gruppe bestehend aus Erdöldestillaten, Kolophonium, Kolophoniumestern, aus Holz gewonnenen Polyterpenen, aus synthetischen Chemikalien gewonnenen Polyterpenen und Kombinationen davon, umfasst, wobei das Klebrigmacherharz einen Erweichungspunkt von wenigstens 80 °C und eine Viskosität von wenigstens 1500 cps bei 360 °F (182 °C) aufweist, wobei die Zusammensetzung eine Viskosität von ungefähr 5000 bis 80000 cps zwischen 350 und 400 °F (177 und 204 °C) aufweist.

2. Zusammensetzung nach Anspruch 1, wobei das Grundpolymer einen Styrolgehalt zwischen ungefähr 15 Gew.-% und ungefähr 45 Gew.-% umfasst.

3. Zusammensetzung nach Anspruch 1 oder 2, wobei das Grundpolymer einen Styrolgehalt zwischen ungefähr 18 Gew.-% und ungefähr 30 Gew.-% umfasst.

4. Zusammensetzung nach einem der vorangehenden Ansprüche, wobei das Grundpolymer eine Schmelzflussrate zwischen ungefähr 5 und ungefähr 200 Gramm pro Minute und eine Shore A-Härte zwischen ungefähr 20 und ungefähr 70 aufweist und bis zu ungefähr 1300 % gestreckt werden kann.

5. Zusammensetzung nach einem der vorangehenden Ansprüche, wobei der Klebrigmacher mit hohem Erweichungspunkt in einer Menge zwischen ungefähr 30 Gew.-% und ungefähr 70 Gew.-% in der Zusammensetzung vorhanden ist.

6. Zusammensetzung nach einem der vorangehenden Ansprüche, außerdem umfassend einen Zusatz mit niedrigem Erweichungspunkt, der einen Erweichungspunkt von weniger als 80 °C und eine Viskosität von weniger als 1000 cps bei 360 °F (182 °C) aufweist und in einer Menge zwischen ungefähr 0 Gew.-% und ungefähr 20 Gew.-% vorhanden ist.

7. Zusammensetzung nach einem der vorangehenden Ansprüche, außerdem umfassend ein Antioxidationsmittel in einer Menge zwischen ungefähr 0,1 Gew.-% und ungefähr 1,0 Gew.-%.

8. Zusammensetzung nach einem der vorangehenden Ansprüche, wobei die Zusammensetzung eine Viskosität von ungefähr 10000 bis 50000 cps zwischen 350 und 385 °F (177 und 196 °C) aufweist.

9. Zusammensetzung nach einem der vorangehenden Ansprüche, wobei die Zusammensetzung als eine Mehrzahl von extrudierten Strängen ausgebildet ist.

10. Zusammensetzung nach einem der Ansprüche 1 bis 8, wobei die Zusammensetzung als eine extrudierte Folie ausgebildet ist.

11. Zusammensetzung nach einem der Ansprüche 1 bis 8, wobei die Zusammensetzung als eine Kombination aus einer Mehrzahl von extrudierten Strängen und einer extrudierten Folie ausgebildet ist.

12. Zusammensetzung nach einem der vorangehenden Ansprüche, wobei die Zusammensetzung durch eine herkömmliche Heißschmelzanlage verarbeitet werden kann.

13. Elastomeres Laminat, umfassend:

   wenigstens eine Deckschicht (facing sheet); und
   eine in einem Schmelztank verarbeitbare elastomere Klebstoffzusammensetzung, die von selbst an der wenigstens einen Deckschicht klebt, wobei die elastomere Klebstoffzusammensetzung wie in einem der Ansprüche 1 bis 8 beansprucht ist.

14. Elastomeres Laminat nach Anspruch 13, wobei die Zusammensetzung als eine extrudierte Folie ausgebildet ist.

15. Elastomeres Laminat nach Anspruch 14, wobei die Zusammensetzung als eine Mehrzahl von extrudierten Strängen ausgebildet ist.

16. Elastomeres Laminat nach Anspruch 15, wobei die Mehrzahl von Strängen auf der wenigstens einen Deckschicht in Abständen von ungefähr 5 bis ungefähr 15 Strängen pro Zoll (2,54 cm) angeordnet sind.

**17.** Elastomeres Laminat nach Anspruch 16, wobei jeder von der Mehrzahl von Strängen einen Durchmesser zwischen ungefähr 0,1 und ungefähr 0,25 Zoll (0,25 und 0,63 cm) aufweist.

**18.** Elastomeres Laminat nach einem der Ansprüche 13 bis 17, wobei die wenigstens eine Deckschicht eine Vliesstofflage, ausgewählt aus einer Spinnvlieslage und einer Schmelzblaslage, umfasst.

**19.** Elastomeres Laminat nach einem der Ansprüche 13 bis 18, wobei die wenigstens eine Deckschicht eine Folie umfasst.

**20.** Elastomeres Laminat nach einem der Ansprüche 13 bis 19, wobei das Laminat ein Flächengewicht zwischen ungefähr 20 und ungefähr 120 Gramm pro Quadratmeter aufweist.

**21.** Elastomeres Laminat nach einem der Ansprüche 13 bis 20, wobei das Laminat ein Dehnung-bis-Stopp-Verhältnis (stretch-to-stop ratio) von wenigstens 50 % aufweist.

**22.** Elastomeres Laminat nach Anspruch 21, wobei das Laminat ein Dehnung-bis-Stopp-Verhältnis von wenigstens 150 % aufweist.

**23.** Elastomeres Laminat nach Anspruch 21, wobei das Laminat ein Dehnung-bis-Stopp-Verhältnis von wenigstens 250 % aufweist.

**24.** Elastomeres Laminat nach einem der Ansprüche 13 bis 23, wobei das Laminat eine Spannung von wenigstens 100 Gramm pro Zoll bei 100 % Dehnung aufweist.

**25.** Elastomeres Laminat nach einem der Ansprüche 13 bis 23, wobei das Laminat eine Spannung von wenigstens 200 Gramm pro Zoll bei 100 % Dehnung aufweist.

**26.** Elastomeres Laminat nach einem der Ansprüche 13 bis 25, wobei das Laminat weniger als 10 % Kriechen nach einer Alterung bei 100 °F während 90 Minuten aufweist.

**27.** Elastomeres Laminat nach einem der Ansprüche 13 bis 26, außerdem umfassend:

ein Kleidungsstück, in dem das elastomere Laminat in eine Struktur des Kleidungsstücks eingearbeitet ist.

**28.** Elastomeres Laminat nach Anspruch 27, wobei das Kleidungsstück ausgewählt ist aus der Gruppe bestehend aus Körperpflegebekleidung, medizinischer Bekleidung und industrieller Arbeitsbekleidung.

**29.** Elastomeres Laminat nach Anspruch 27, wobei das Kleidungsstück ausgewählt ist aus der Gruppe bestehend aus Windeln, Trainingshosen, Badeanzügen, absorbierenden Unterhosen, Inkontinenzprodukten für Erwachsene, Hygieneprodukten für Frauen, medizinischen Schutzanzügen, medizinischen Operationsgewändern, Kopfbedeckungen, Handschuhen, Vorhängen, Gesichtsmasken, Labormänteln und Overalls.

**30.** Verfahren zum Herstellen einer elastomeren Klebstoffzusammensetzung, umfassend;

das Bilden einer Festphasenzusammensetzung wie in einem der Ansprüche 1 bis 8 beansprucht;
das Bilden einer Flüssigphasenzusammensetzung durch Erwärmen der Festphasenzusammensetzung;
das Bilden einer Folie durch Extrudieren der Flüssigphasenzusammensetzung auf eine Kühlwalze: und
das Abziehen der Folie von der Kühlwalze, während die Folie gestreckt wird.

**31.** Verfahren nach Anspruch 30, umfassend das Extrudieren der Flüssigphasenzusam-mensetzung durch eine Schlitz-Beschichtungsdüse auf die Kühlwalze.

**32.** Verfahren nach Anspruch 30 oder 31, umfassend das Strecken der Folie mit einem Ausstoß zwischen ungefähr 50 und ungefähr 120 Gramm pro Quadratmeter vor dem Strecken, aus der Schlitz-Beschichtungsdüse.

**33.** Verfahren nach Anspruch 30, 31 oder 32, umfassend das Extrudieren der Flüssigphasenzusammensetzung durch eine Extruderdüse auf die Kühlwalze.

34. Verfahren nach einem der Ansprüche 30 bis 33, umfassend das Strecken der Folie mit einem Ausstoß (Output) zwischen ungefähr 50 und ungefähr 120 Gramm pro Quadratmeter, vor dem Strecken, aus der Extruderdüse.

35. Verfahren nach einem der Ansprüche 30 bis 34, umfassend das Strecken der Folie bis ungefähr 1200 %, vorzugsweise bis ungefähr 1000 %, vorzugsweise bis ungefähr 700 %, vorzugsweise bis ungefähr 500 %.

36. Verfahren nach einem der Ansprüche 30 bis 35, außerdem umfassend das Laminieren der gestreckten Folie zwischen zwei Deckschichten.

37. Verfahren nach Anspruch 36, wobei wenigstens eine der Deckschichten eine Vliesstofflage umfasst.

38. Verfahren nach Anspruch 36, wobei wenigstens eine der Deckschichten eine Folie umfasst.

39. Verfahren nach einem der Ansprüche 30 bis 38, wobei die Flüssigphasenzusammensetzung durch Erwärmen der Festphasenzusammensetzung auf eine Temperatur zwischen ungefähr 350 und ungefähr 400 °F (176 und 204 °C) gebildet wird.

40. Verfahren nach einem der Ansprüche 30 bis 39, wobei die Kühlwalze auf eine Temperatur zwischen ungefähr 10 und ungefähr 50 °C eingestellt ist, während die Folie gebildet wird.

41. Verfahren nach einem der Ansprüche 30 bis 40, umfassend das Ausbilden bzw. Formen der Folie zu einer Mehrzahl von extrudierten Strängen.

42. Verfahren zum Herstellen eines elastischen Stranglaminats, umfassend:

   das Erwärmen einer elastomeren Klebstoffzusammensetzung wie in einem der Ansprüche 1 bis 7 beansprucht in einer Heißschmelzanlage;
   das Verarbeiten der elastomeren Klebstoffzusammensetzung durch eine Strangdüse auf eine Kohlwalze, um eine Mehrzahl von elastischen Strängen zu bilden;
   das Strecken der Mehrzahl von elastischen Strängen zwischen der Kühlwalze und einer Andruckwalze; und
   das Selbstankleben der Mehrzahl von elastischen Strängen an wenigstens eine Deckschicht.

43. Verfahren nach Anspruch 42, umfassend das Strecken der Mehrzahl von elastischen Strängen zwischen ungefähr 200 % und ungefähr 1200 %.

44. Verfahren nach Anspruch 42 oder 43, umfassend das Verarbeiten der elastomeren Klebstoffzusammensetzung durch die Strangdüse mit einem Ausstoß zwischen ungefähr 20 und ungefähr 150 Gramm pro Quadratmeter vor dem Strecken.

45. Verfahren nach einem der Ansprüche 42 bis 44, wobei die wenigstens eine Deckschicht eine Folie umfasst.

46. Verfahren nach Anspruch 45, außerdem umfassend das Verarbeiten der Folie, während gleichzeitig die elastomere Klebstoffzusammensetzung verarbeitet wird.

47. Verfahren nach einem der Ansprüche 42 bis 46, wobei die wenigstens eine Deckschicht eine Vliesstofflage, ausgewählt aus einer Spinnvlieslage und einer Schmelzblaslage, umfasst.

48. Verfahren nach einem der Ansprüche 42 bis 47, außerdem umfassend das Einstellen einer Auftragmenge (add-on level) der elastomeren Klebstoffzusammensetzung, um die Spannung in dem elastischen Stranglaminat zu variieren.

49. Verfahren nach einem der Ansprüche 42 bis 48, außerdem umfassend das Einstellen eines Streckverhältnisses der Mehrzahl von elastischen Strängen, um die Spannung in dem elastischen Stranglaminat zu variieren.

50. Verfahren nach einem der Ansprüche 42 bis 49, wobei die Strangdüse Löcher mit variierendem Durchmesser umfasst.

**Revendications**

1. Composition adhésive élastomère, **caractérisée par** :

   un polymère de base, le polymère de base étant présent dans la composition en une quantité comprise entre environ 30% et environ 75% en poids, le polymère de base comprenant au moins un élément du groupe consistant en le copolymère séquencé polystyrène-polyéthylène-polypropylène-polystyrène (SEPS), le styrène-isoprène-styrène (SIS), le copolymère séquencé styrène-butadiène-styrène (SBS), le polyuréthane thermoplastique, le copolymère éthylène-propylène-diène (EPDM), et leurs combinaisons ;
   une résine à haut point de ramollissement, améliorant la pégosité, la résine à haut point de ramollissement, améliorant la pégosité, comprenant au moins un type d'hydrocarbure sélectionné dans le groupe consistant en les distillats de pétrole, la colophane, les esters de colophane, les polyterpènes dérivés du bois, les polyterpènes dérivés de produits chimiques de synthèse, et leurs combinaisons, la résine favorisant la pégosité ayant un point de ramollissement d'au moins 80° Celsius et une viscosité d'au moins 1500 cps à 360° Fahrenheit (182°C), la composition ayant une viscosité comprise entre environ 5 000 et 80 000 cps à une température comprise entre 350 et 400° Fahrenheit (entre 177 et 204° C).

2. Composition selon la revendication 1, dans laquelle le polymère de base a une teneur en styrène comprise entre environ 15% et environ 45% en poids.

3. Composition selon la revendication 1 ou 2, dans laquelle le polymère de base a une teneur en styrène comprise entre environ 18% et environ 30% en poids.

4. Composition selon l'une quelconque des revendications précédentes, dans laquelle le polymère de base a un débit d'écoulement à l'état fondu compris entre environ 5 et environ 200 grammes par minute, une dureté Shore A comprise entre environ 20 et environ 70, la composition pouvant être étirée jusqu'à environ 1300%.

5. Composition selon l'une quelconque des revendications précédentes, dans laquelle la résine à haut point de ramollissement, favorisant la pégosité, est présente dans la composition en une quantité comprise entre environ 30% et environ 70% en poids.

6. Composition selon l'une quelconque des revendications précédentes, comprenant en outre un additif à bas point de ramollissement ayant un point de ramollissement inférieur à 80° Celsius et une viscosité inférieure à 1000 cps à 360° Fahrenheit (182°C), présent en une quantité comprise entre environ 0% et environ 20% en poids.

7. Composition selon l'une quelconque des revendications précédentes, comprenant en outre un antioxydant en une quantité comprise entre environ 0,1% et environ 1,0% en poids.

8. Composition selon l'une quelconque des revendications précédentes, la composition ayant une viscosité comprise entre environ 10 000 et 50 000 cps à une température comprise entre 350 et 385° Fahrenheit (entre 177 et 196°C) .

9. Composition selon l'une quelconque des revendications précédentes, la composition étant formée en tant que pluralité de brins extrudés.

10. Composition selon l'une quelconque des revendications 1 à 8, la composition étant formée en tant que film extrudé.

11. Composition selon l'une quelconque des revendications 1 à 8, la composition étant formée d'une combinaison de pluralité de brins extrudés et d'un film extrudé.

12. Composition selon l'une quelconque des revendications précédentes, la composition pouvant être traitée au moyen d'un équipement classique pour adhésif thermofusible.

13. Laminé élastomère, comprenant :

   au moins une feuille de façade ; et
   une composition adhésive élastomère pouvant être traitée en cuve de fusion, auto-collée à ladite au moins une feuille de façade, la composition adhésive élastomère étant telle que revendiquée dans l'une quelconque des revendications 1 à 8.

**14.** Laminé élastomère selon la revendication 13, dans lequel la composition est formée en tant que film extrudé.

**15.** Laminé élastomère selon la revendication 14, dans lequel la composition est formée en tant que pluralité de brins extrudés.

**16.** Laminé élastomère selon la revendication 15, dans lequel les brins de la pluralité sont espacés les uns des autres sur ladite au moins une feuille de façade à raison d'environ 5 à environ 15 brins par pouce (2,54 cm).

**17.** Laminé élastomère selon la revendication 16, dans lequel chacun des brins de la pluralité a un diamètre compris entre environ 0,1 et environ 0,25 pouce (entre 0,25 et 0,63 cm).

**18.** Laminé élastomère selon l'une quelconque des revendications 13 à 17, dans lequel ladite au moins une feuille de façade comprend un voile non-tissé sélectionné entre un voile obtenu par filage-nappage et un voile obtenu par extrusion-soufflage.

**19.** Laminé élastomère selon l'une quelconque des revendications 13 à 18, dans lequel ladite au moins une feuille de façade comprend un film.

**20.** Laminé élastomère selon l'une quelconque des revendications 13 à 19, dans lequel le laminé a une masse surfacique comprise entre environ 20 et environ 120 grammes par mètre carré.

**21.** Laminé élastomère selon l'une quelconque des revendications 13 à 20, le laminé ayant un rapport étirage jusqu'à la limite d'au moins 50%.

**22.** Laminé élastomère selon la revendication 21, le laminé ayant un rapport étirage jusqu'à la limite d'au moins 150%.

**23.** Laminé élastomère selon la revendication 21, le laminé ayant un rapport étirage jusqu'à la limite d'au moins 250%.

**24.** Laminé élastomère selon l'une quelconque des revendications 13 à 23, le laminé ayant une tension d'au moins 100 grammes par pouce à 100% d'allongement.

**25.** Laminé élastomère selon l'une quelconque des revendications 13 à 23, le laminé ayant une tension d'au moins 200 grammes par pouce à 100% d'allongement.

**26.** Laminé élastomère selon l'une quelconque des revendications 13 à 25, le laminé manifestant un fluage inférieur à 10% après un vieillissement à 100° Fahrenheit pendant 90 minutes.

**27.** Laminé élastomère selon l'une quelconque des revendications 13 à 26, comprenant en outre :

un vêtement incorporant le laminé élastomère dans une structure du vêtement.

**28.** Laminé élastomère selon la revendication 27, dans lequel le vêtement est sélectionné dans le groupe consistant en les vêtements d'hygiène intime, les vêtements médicaux et les vêtements de travail pour l'industrie.

**29.** Laminé élastomère selon la revendication 27, dans lequel le vêtement est sélectionné dans le groupe consistant en les couches-culottes, les culottes d'apprentissage de la propreté, les maillots de bain, les sous-vêtements absorbants, les produits pour l'incontinence adulte, les produits d'hygiène féminine, les blouses médicales de protection, les casaques médico-chirurgicales, les bonnets, les gants, les champs opératoires, les masques faciaux, les blouses de laboratoire, et les combinaisons.

**30.** Procédé de fabrication d'une composition adhésive élastomère comprenant ;
la formation d'une composition en phase solide telle que revendiquée dans l'une quelconque des revendications 1 à 8 ;
la formation d'une composition en phase liquide par chauffage de la composition en phase solide ;
la formation d'un film par extrusion, sur un rouleau refroidisseur, de la composition en phase liquide ; et
la séparation par pelage du film, depuis le rouleau refroidisseur, tout en étirant le film.

**31.** Procédé selon la revendication 30, comprenant l'extrusion de la composition en phase liquide, sur le rouleau refroidisseur, au travers d'une filière d'enduction à fente.

32. Procédé selon la revendication 30 ou 31, comprenant l'étirage du film à un débit compris entre environ 50 et environ 120 grammes par mètre carré avant l'étirage, depuis la filière d'enduction à fente.

33. Procédé selon la revendication 30, 31 ou 32, comprenant l'extrusion de la composition en phase liquide, sur le rouleau refroidisseur, au travers d'une filière d'extrusion.

34. Procédé selon l'une quelconque des revendications 30 à 33, comprenant l'étirage du film à un débit d'environ 50 à environ 120 grammes par mètre carré, avant l'étirage, depuis la filière d'extrusion.

35. Procédé selon l'une quelconque des revendications 30 à 34, comprenant l'étirage du film jusqu'à environ 1200%, de préférence jusqu'à environ 1000%, de préférence jusqu'à environ 700%, et de préférence jusqu'à environ 500%.

36. Procédé selon l'une quelconque des revendications 30 à 35, comprenant en outre la stratification, entre deux feuilles de façade, du film étiré.

37. Procédé selon la revendication 36, dans lequel l'une au moins des feuilles de façade comprend un voile non-tissé.

38. Procédé selon la revendication 36, dans lequel l'une au moins des feuilles de façade comprend un film.

39. Procédé selon l'une quelconque des revendications 30 à 38, dans lequel la composition en phase liquide est formée par chauffage de la composition en phase solide à une température comprise entre environ 350 et environ 400° Fahrenheit (entre 176 et 204°.).

40. Procédé selon l'une quelconque des revendications 30 à 39, dans lequel le rouleau refroidisseur est réglé à une température comprise entre environ 10 et environ 50° Celsius tandis que le film est en cours de formation.

41. Procédé selon l'une quelconque des revendications 30 à 40, comprenant la transformation du film en une pluralité de brins extrudés.

42. Procédé de fabrication d'un laminé de brins élastiques, comprenant :

le chauffage d'une composition adhésive élastomère telle que revendiquée dans l'une quelconque des revendications 1 à 7 dans un équipement pour adhésif thermofusible ;
le traitement de la composition adhésive élastomère au travers d'une filière à brins, sur un rouleau refroidisseur, pour former une pluralité de brins élastiques ;
l'étirage de la pluralité de brins élastiques entre le rouleau refroidisseur et un espace-pinceur entre rouleaux ; et
l'auto-adhérence de la pluralité de brins élastiques à au moins une feuille de façade.

43. Procédé selon la revendication 42, comprenant l'étirage de la pluralité de brins élastiques entre environ 200% et environ 1200%.

44. Procédé selon la revendication 42 ou 43, comprenant le traitement de la composition adhésive élastomère au travers de la filière à brins à un débit compris entre environ 20 et environ 150 grammes par mètre carré, avant l'étirage.

45. Procédé selon l'une quelconque des revendications 42 à 44, dans lequel ladite au moins une feuille de façade comprend un film.

46. Procédé selon la revendication 45, comprenant en outre le traitement du film tout en traitant simultanément la composition adhésive élastomère.

47. Procédé selon l'une quelconque des revendications 42 à 46, dans lequel ladite au moins une feuille de façade comprend un voile non-tissé sélectionné entre un voile obtenu par filage-nappage et un voile obtenu par extrusion-soufflage.

48. Procédé selon l'une quelconque des revendications 42 à 47, comprenant en outre le réglage d'un taux d'adjonction de la composition adhésive élastomère pour faire varier la tension du laminé de brins élastiques.

**49.** Procédé selon l'une quelconque des revendications 42 à 48, comprenant en outre le réglage d'un rapport d'étirage de la pluralité de brins élastiques pour faire varier la tension du laminé de brins élastiques.

**50.** Procédé selon l'une quelconque des revendications 42 à 49, dans lequel la filière à brins comprend des trous de diamètre variable.

20

30

2

2

2

**FIG. 1**

22    22    22    22    20

**FIG. 2**

FIG. 3

FIG. 4

FIG. 5

FIG. 6

FIG. 7

FIG. 8

FIG. 9

FIG. 10

FIG. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4698242 A
- US 4543099 A
- US 5536563 A
- US 4789699 A
- US 3849241 A, Butin
- US 4340563 A, Appel
- US 3692618 A, Dorschner
- US 3802817 A, Matsuki
- US 3338992 A
- US 3341394 A, Kinney
- US 3502763 A, Hartmann
- US 3502538 A, Petersen
- US 3542615 A, Dobo
- US 5336552 A, Strack